(19) Europäisches Patentamt
European Patent Office
Office européen des brevets

(11) Veröffentlichungsnummer: **0 101 006**
**A1**

# (12) EUROPÄISCHE PATENTANMELDUNG

(21) Anmeldenummer: 83107633.6

(22) Anmeldetag: 03.08.83

(51) Int. Cl.$^3$: C 07 C 125/065
A 01 N 47/12, A 01 N 47/18

(30) Priorität: 14.08.82 DE 3230295

(43) Veröffentlichungstag der Anmeldung: 22.02.84 Patentblatt 84/8

(84) Benannte Vertragsstaaten: AT BE CH DE FR GB IT LI NL SE

(71) Anmelder: BAYER AG
Zentralbereich Patente, Marken und Lizenzen
D-5090 Leverkusen 1, Bayerwerk(DE)

(72) Erfinder: Heywang, Gerhard, Dr.
Nittumer Weg 4
D-5060 Bergisch-Gladbach 2(DE)

(72) Erfinder: Kühle, Engelbert, Dr.
von Bodelschwingh-Strasse 42
D-5060 Bergisch-Gladbach 2(DE)

(72) Erfinder: Behrenz, Wolfgang, Dr.
Untergruendemich 14
D-5063 Overath(DE)

## (54) Carbamidsäureester. Verfahren zu ihrer Herstellung und ihre Verwendung als Schädlingsbekämpfungsmittel.

(57) Die vorliegende Erfindung betrifft die teilweise neuen Carbamidsäureester der allgemeinen Formel (I)

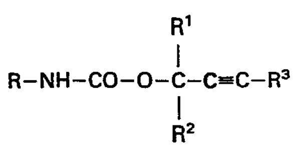

(I)

in welcher

R für gegebenenfalls substituiertes Alkyl mit 4 und mehr Kohlenstoffatomen oder gegebenenfalls substituiertes Cycloalkyl steht und

$R^1$, $R^2$ und $R^3$ gleich oder verschieden sind und einzeln für Wasserstoff oder für gegebenenfalls substituiertes Alkyl stehen, welche

als Synergisten in Schädlingsbekämpfungsmitteln verwendet werden können, die zusätzlich Arthropodizide enthalten, welche vorzugsweise gegen Insekten und Spinnentiere, insbesondere gegen Insekten wirksam sind.

EP 0 101 006 A1

Croydon Printing Company Ltd

BAYER AKTIENGESELLSCHAFT 5090 Leverkusen, Bayerwerk

Zentralbereich
Patente, Marken und Lizenzen S/by-c
I/III

# Carbamidsäureester, Verfahren zu ihrer Herstellung und ihre Verwendung als Schädlingsbekämpfungsmittel

Die vorliegende Erfindung betrifft Carbamidsäureester, Verfahren zu ihrer Herstellung sowie ihre Verwendung als Schädlingsbekämpfungsmittel, vorzugsweise zur Bekämpfung von Arthropoden, insbesondere von Insekten, Milben und Spinnentieren.

Synergistische Mischungen von insektiziden Wirkstoffen, z.B. von Pyrethroiden mit bestimmten Methylendioxyphenyl-Derivaten, z.B. Piperonylbutoxid als Synergisten sind bereits bekannt geworden (vgl. z.B. K. Naumann, Chemie der Pflanzenschutz- und Schädlingsbekämpfungsmittel, Springer Verlag Berlin, Band 7 (1981) Seiten 3-6). Ferner sind bestimmte N-Arylcarbamidsäurealkinylester (vgl. DE-A 2 041 986) und bestimmte N-Alkylcarbamidsäurealkinylester (vgl. BE-A- 633 594) als Synergisten beschrieben worden. Die Wirksamkeit dieser Verbindungen ist jedoch unter den in der Praxis auftretenden Bedingungen nicht immer voll befriedigend.

Es wurde gefunden, daß die Carbamidsäureester der allgemeinen Formel (I)

$$R\text{-}NH\text{-}CO\text{-}O\text{-}\underset{R^2}{\overset{R^1}{C}}\text{-}C\equiv C\text{-}R^3 \qquad (I)\ .$$

in welcher

R für gegebenenfalls substituiertes Alkyl mit 4 oder mehr Kohlenstoffatomen oder gegebenenfalls substituiertes Cycloalkyl steht und

$R^1$, $R^2$ und $R^3$ gleich oder verschieden sind und einzeln für Wasserstoff oder für gegebenenfalls substituiertes Alkyl stehen,

als Synergisten in Schädlingsbekämpfungsmitteln verwendet werden können, die zusätzlich Arthropodizide enthalten, welche vorzugsweise gegen Insekten und Spinnentiere, insbesondere gegen Insekten wirksam sind.

Als Arthropodizide (gegen Arthropoden wirksame Stoffe) kommen praktisch alle üblichen Wirkstoffe in Frage (vgl. z.B. K.H. Büchel, Pflanzenschutz und Schädlingsbekämpfungsmittel, Thieme Verlag Stuttgart, 1977, und Farm Chemicals Handbook 1979, Meister Publishing Co, Willougby, 1979).

Die Carbamidsäureester der allgemeinen Formel (Ia)

$$R\text{-}NH\text{-}CO\text{-}O\text{-}\underset{R^2}{\overset{R^1}{C}}\text{-}C\equiv C\text{-}R^3 \qquad (Ia)$$

in welcher

R für gegebenenfalls substituiertes Alkyl mit 4 oder mehr Kohlenstoffatomen oder gegebenenfalls substituiertes Cycloalkyl steht und

$R^1$, $R^2$ und $R^3$ gleich oder verschieden sind und einzeln für Wasserstoff oder für gegebenenfalls substituiertes Alkyl stehen,

ausgenommen die Verbindung in welcher R für n-Butyl und $R^1$, $R^2$ und $R^3$ für Wasserstoff stehen, sind neu und stellen einen Teil der Erfindung dar.

Die Verbindung der Formel (Ia) mit R = n-Butyl und $R^1$, $R^2$ und $R^3$ = Wasserstoff, wurde aus der EP-A 14032 als Synthesezwischenprodukt (ohne die Nennung einer direkten Verwendbarkeit) bekannt.

Weiterhin wurde gefunden, daß man die neuen Carbamidsäureester der allgemeinen Formel (Ia)

$$R\text{-}NH\text{-}CO\text{-}O\text{-}\underset{R^2}{\overset{R^1}{C}}\text{-}C\equiv C\text{-}R^3 \qquad (Ia)$$

in welcher

R für gegebenenfalls substituiertes Alkyl mit 4 oder mehr Kohlenstoffatomen oder gegebenenfalls substituiertes Cycloalkyl steht und

$R^1$, $R^2$ und $R^3$ gleich oder verschieden sind und einzeln für Wasserstoff oder für gegebenenfalls substituiertes Alkyl stehen,

ausgenommen die Verbindung in welcher R für n-Butyl und $R^1$, $R^2$ und $R^3$ für Wasserstoff stehen, erhält, wenn man Alkylisocyanate der allgemeinen Formel (II)

$$R - NCO \qquad (II)$$

in welcher

R die oben angegebene Bedeutung hat

mit Alkinylalkoholen der allgemeinen Formel (III)

$$HO-\overset{R^1}{\underset{R^2}{C}}-C{\equiv}C-R^3 \qquad (III)$$

in welcher

$R^1$, $R^2$ und $R^3$ die oben angegebene Bedeutung haben,

gegebenenfalls in Gegenwart eines aprotischen Verdünnungsmittels und gegebenenfalls in Gegenwart

eines Katalysators bei Temperaturen zwischen 0 und 120°C umsetzt (die Verbindung mit R=n-$C_4H_9$ und $R^1$, $R^2$ und $R^3$ = H, kann nach dem gleichen Verfahren erhalten werden).

Als gegebenenfalls substituiertes Alkyl R steht vorzugsweise geradkettiges oder verzweigtes gegebenenfalls substituiertes Alkyl mit 4-20, insbesondere 4-12 und besonders bevorzugt mit 4-8 Kohlenstoffatomen. Beispielhaft seien gegebenenfalls substituiertes n-, iso- und tert.-Butyl, Neopentyl und Hexyl genannt. Besonders hervorgehoben sei der Neopentylrest.

Als gegebenenfalls substituiertes Cycloalkyl R steht mono-, bi- und tricyclisches Cycloalkyl mit vorzugsweise 4 bis 10, insbesondere 4, 5 oder 6 Kohlenstoffatomen. Beispielhaft seien gegebenenfalls substituiertes Cyclobutyl, Cyclopentyl, Cyclohexyl, Cycloheptyl, Bicyclo[2,2,1]-heptyl, Bicyclo[2,2,2]-octyl und Adamantyl genannt. Besonders bevorzugt wird der gegebenenfalls substituierte Cyclohexylrest.

Als gegebenenfalls substituiertes Alkyl $R^1$, $R^2$ und $R^3$ steht geradkettiges oder verzweigtes gegebenenfalls substituiertes Alkyl mit vorzugsweise 1-8, insbesondere 1-5 und besonders bevorzugt 1-3 Kohlenstoffatomen. Beispielhaft seien gegebenenfalls substituiertes Methyl, Ethyl, n- und i-Propyl sowie n-, iso- und tert.-Butyl genannt, wobei diese Reste bevorzugt unsubstituiert sind.

Die in der Definition von R genannten Alkyl- und Cycloalkylreste können einen oder mehrere, vorzugsweise 1 bis 3, insbesondere 1 oder 2 gleiche oder verschiedene Substituenten tragen. Als Substituenten seien beispielhaft aufgeführt:

Alkoxy mit vorzugsweise 1 bis 4, insbesondere 1 oder 2 Kohlenstoffatomen, wie Methoxy, Ethoxy, n- und iso-Propyloxy und n-, iso- und tert.-Butyloxy; Alkylthio mit vorzugsweise 1 bis 4, insbesondere 1 oder 2 Kohlenstoffatomen, wie Methylthio, Ethylthio, n- und iso-Propylthio und n-, iso- und tert.-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl; Halogen, vorzugsweise Fluor, Chlor, Brom und Jod, insbesondere Chlor und Brom; Cyano; Nitro; Alkoxycarbonyl mit vorzugsweise 2 bis 4, insbesondere 2 oder 3 Kohlenstoffatomen, wie Methoxycarbonyl und Ethoxycarbonyl sowie Phenoxybenzyloxycarbonyl.

Die in der Definition von $R^1$ und $R^2$ genannten Alkylreste können einen oder mehrere, vorzugsweise 1 bis 3, insbesondere 1 oder 2 gleiche oder verschiedene Substituenten tragen. Als Substituenten seien beispielsweise aufgeführt:

Alkoxy mit vorzugsweise 1 bis 4, insbesondere 1 oder 2 Kohlenstoffatomen, wie Methoxy, Ethoxy, n- und iso-Propyloxy und n-, iso- und tert.-Butyloxy; Alkylthio mit vorzugsweise 1 bis 4, insbesondere 1 oder 2 Kohlenstoffatomen, wie Methylthio, Ethylthio, n- und iso-Propylthio und n-, iso- und tert.-Butylthio; Halogenalkyl mit vorzugsweise 1 bis 4, insbesondere 1 oder 2 Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl; Halogen, vorzugsweise Fluor, Chlor, Brom und Jod, insbesondere Chlor und Brom; Cyano; Nitro; Alkoxycarbonyl mit vorzugsweise 2 bis 4, insbesondere 2 oder 3 Kohlenstoffatomen, wie Methoxycarbonyl und Ethoxycarbonyl.

Der in der Definition von $R^3$ genannte Alkylrest kann einen oder mehrere, vorzugsweise 1 bis 3, insbesondere 1 oder 2 gleiche oder verschiedene Substituenten tragen. Als Substituenten seien beispielhaft aufgeführt:

Alkoxy mit vorzugsweise 1 bis 4, insbesondere 1 oder 2 Kohlenstoffatomen, wie Methoxy, Ethoxy, n- und iso-Propyloxy und n-, iso- und tert.-Butyloxy; Alkylthio mit vorzugsweise 1 bis 4, insbesondere 1 oder 2 Kohlenstoffatomen, wie Methylthio, Ethylthio, n- und iso-Propylthio und n-, iso- und tert.-Butylthio; Halogenalkyl mit vorzugsweise 1 bis 4, insbesondere 1 oder 2

Kohlenstoffatomen und vorzugsweise 1 bis 5, insbesondere 1 bis 3 Halogenatomen, wobei die Halogenatome gleich oder verschieden sind und als Halogenatome, vorzugsweise Fluor, Chlor oder Brom, insbesondere Fluor stehen, wie Trifluormethyl; Halogen, vorzugsweise Fluor, Chlor, Brom und Jod, insbesondere Chlor und Brom; Cyano; Nitro; Alkoxycarbonyl mit vorzugsweise 2 bis 4, insbesondere 2 oder 3 Kohlenstoffatomen, wie Methoxycarbonyl und Ethoxycarbonyl.

Als bevorzugter Substituent des Alkylrestes R sei Halogen (Fluor, Chlor und Brom, vorzugsweise Chlor) und des Cycloalkylrestes R sei Halogenalkyl (vorzugsweise Trifluormethyl) genannt.

Vorzugsweise sind die Reste R, $R^1$ und $R^2$ unsubstituiert.

Bevorzugt werden die Carbamidsäureester der allgemeinen Formel (I), in welcher

R für einen gegebenenfalls durch Alkoxy ($C_1$-$C_4$), Halogen, Cyano, Nitro und/oder Trifluormethyl substituierten Alkylrest mit 4-12 Kohlenstoffatomen oder für einen gegebenenfalls durch Alkoxy ($C_1$-$C_4$), Halogen, Cyano, Nitro und/oder Trifluormethyl substituierten Cycloalkylrest mit 4-10 Kohlenstoffatomen steht und

$R^1$,$R^2$,$R^3$ gleich oder verschieden sind und einzeln für Wasserstoff oder für einen gegebenenfalls durch Halogen, Cyano, $C_1$-$C_4$-Alkoxy, $C_1$-$C_4$-

Alkylthio oder Trifluormethyl substituierter Alkylrest mit 1-2 Kohlenstoffatomen stehen.

Die Erfindung betrifft besonders bevorzugt Carbamidsäureester der allgemeinen Formel (I), in welcher

R für einen gegebenenfalls durch Fluor, Chlor oder Cyano substituierten Alkylrest mit 4-8 Kohlenstoffatomen oder für einen durch Fluor, Chlor, Cyano oder Trifluormethyl substituierten Cycloalkylrest mit 5-6 Kohlenstoffatomen steht und

$R^1, R^2, R^3$ gleich oder verschieden sind und einzeln für Wasserstoff oder für einen gegebenenfalls durch Fluor, Chlor, Methoxy oder Methylthio substituierten Alkylrest mit 1 bis 3 Kohlenstoffatomen, stehen.

Ganz besonders hervorgehoben werden die Carbamidsäureester der allgemeinen Formel (I), in welcher

R für gegebenenfalls durch Chlor oder Cyano substituiertes Alkyl mit 4 bis 6 Kohlenstoffatomen oder für gegebenenfalls durch Trifluormethyl substituiertes Cyclohexyl steht und

$R^1, R^2$ und $R^3$ für Wasserstoff stehen.

Als neue Carbamidsäureester der Formel (Ia) werden besonders die Verbindungen bevorzugt, in welchen

R für Neopentyl oder für gegebenenfalls durch Trifluormethyl substituiertes Cyclohexyl steht und

$R^1$, $R^2$ und $R^3$ für Wasserstoff stehen.

Verwendet man beispielsweise Neopentylisocyanat und Propargylalkohol als Ausgangsstoffe, so kann das erfindungsgemäße Verfahren durch folgendes Formelschema erläutert werden:

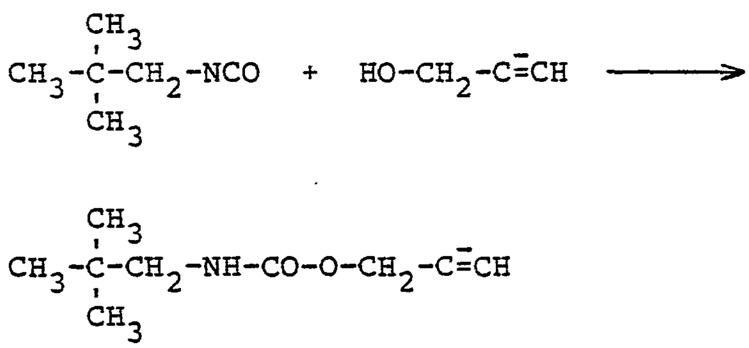

Beim erfindungsgemäßen Verfahren können die Ausgangsmaterialien vorzugsweise in äquimolaren Mengen eingesetzt werden. Es ist jedoch auch möglich eine der Komponenten gleichzeitig als Verdünnungsmittel zu verwenden.

Beim erfindungsgemäßen Verfahren sind praktisch alle aprotischen Lösungsmittel als Verdünnungsmittel geeignet. Bevorzugt seien hier Ketone, wie Aceton, gegebenenfalls halogenierte aliphatische oder aromatische Kohlenwasserstoffe, wie Methylenchlorid oder Toluol, Nitrile, wie Acetonitril sowie deren Gemische genannt. Es ist jedoch auch möglich ohne Verdünnungsmittel zu arbeiten.

Als Katalysatoren werden dem Reaktionsgemisch gegebenenfalls Triethylamin, Diazabicyclooctan (DABCO) oder Dibutylzinnlaurat zugesetzt.

Die Reaktionstemperatur wird beim erfindungsgemäßen Verfahren zwischen etwa 0 und 120°C, vorzugsweise zwischen etwa 0 und 60°C gehalten. Das Verfahren wird vorzugsweise bei Normaldruck durchgeführt.

Die Ausgangsverbindungen der Formel (II) sind bekannt und/oder können in üblicher Weise nach an sich bekannten Verfahren und Methoden hergestellt werden (vgl. z.B. Methoden der organischen Chemie (Houben-Weyl-Müller) 4. Auflage, Band 8 (1952) S. 119 ff.).

Die Ausgangsverbindungen der Formel (III) sind bekannt und/oder können in üblicher Weise nach an sich bekannten Verfahren hergestellt werden (Vergl. W. Reppe J.Liebigs Ann. Chem. 569, 1 (1955), W. Ried, angew. Chem. 76, 973 (1964), W. Ried et al. Chem. Ber. 98, 245 (1965)).

Die Carbamidsäureester der allgemeinen Formel (I) weisen in Mischung mit Stoffen beliebiger Konstitution, welche gegen Arthropoden wirksam sind, starke synergistische Wirkungen auf, welche ihre Verwendung in Schädlingsbekämpfungsmitteln ermöglicht. (Im folgenden soll der Hinweis auf die allgemeine Formel (I) jeweils auch die Verbindungen der allgemeinen Formel (Ia) einschließen).

Bevorzugt werden die Carbamidsäureester der allgemeinen Formel (I) zusammen mit Arthropodiziden

1. Carbaminsäureestern und/oder

2. Carbonsäureestern einschließlich der natürlichen sowie synthetischen Pyrethroide und/oder

3. Phosphorverbindungen, wie Phosphorsäure- und Phosphonsäureestern, einschließlich der Thio- und Dithioverbindungen und/oder

4. Halogen-(cyclo)-alkanen, wie z.B. Hexachlorcyclohexan

verwendet.

Überraschenderweise ist die Wirkung der neuen erfindungsgemäßen Wirkstoffkombinationen gegen Arthropoden wesentlich höher als die Wirkung der Einzelkomponenten bzw. die Summe der Wirkungen der Einzelkomponenten. Sie ist ferner wesentlich höher als die Wirkung von Wirkstoffkombinationen mit dem bekannten handelsüblichen Synergisten Piperonylbutoxid. Die erfindungsgemäß verwendbaren Carbamidsäureester zeigen außerdem ausgezeichnete synergistische Wirksamkeit nicht nur bei einer Wirkstoffklasse, sondern bei Wirkstoffen aus den verschiedensten chemischen Stoffgruppen.

Die synergistische Wirkung der Verbindungen der allgemeinen Formel (I) zeigt sich besonders bevorzugt bei

1) Carbamidsäureestern der Formel (IV)

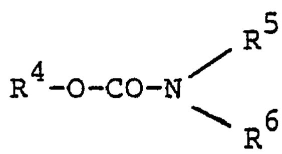

(IV)

in welcher

$R^4$ für einen gegebenenfalls substituierten carbocyclischen oder heterocyclischen aromatischen Rest oder für einen gegebenenfalls substituierten Oximrest steht (wobei die weiter unten erläuterten Reste $R^4$ bevorzugt werden),

$R^5$ für $C_1$-$C_4$-Alkyl steht und

$R^6$ für Wasserstoff, $C_1$-$C_4$-Alkyl oder für einen Rest Y steht, wobei

Y für den Rest $-CO-R^7$ steht, worin

$R^7$ für Halogen, $C_1$-$C_4$-Alkyl, $C_1$-$C_4$-Alkoxy, $C_3$-$C_5$-Alkenoxy, $C_3$-$C_5$-Alkinoxy, $C_1$-$C_4$-Alkylthio, $C_1$-$C_4$-Alkyl-amino, Di-$C_1$-$C_4$-Alkylamino, $C_1$-$C_4$-Alkyl-hydroxylamino,

für gegebenenfalls durch Halogen, Nitro, Cyano, Trifluormethyl, $C_1$-$C_4$-Alkyl, $C_1$-$C_4$-Alkoxy, $C_1$-$C_4$-Alkylendioxy, $C_1$-$C_4$-Alkylthio, $C_1$-$C_4$-Alkoxy-carbonyl substituiertes Phenoxy, Phenylthio oder Phenylamino, für 2,3-Dihydro-2,2-dimethyl-7-benzofuranyl oder für den Rest

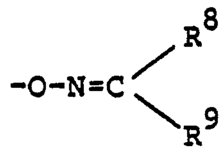

steht, worin

$R^8$ für Wasserstoff, $C_1$-$C_4$-Alkyl oder Di-$C_1$-$C_4$-alkylamino-carbonyl steht und

$R^9$ für $C_1$-$C_4$-Alkyl, $C_1$-$C_4$-Alkylthio, Cyano-$C_1$-$C_4$-alkylthio, $C_1$-$C_4$-Alkylthio-$C_1$-$C_4$-alkyl steht,

oder die beiden Reste $R^8$ und $R^9$ zusammen für gegebenenfalls durch Sauerstoff, Schwefel, SO oder $SO_2$ unterbrochenes $C_2$-$C_8$-Alkandiyl stehen, oder

in welcher

Y für den Rest $-S_n(O)_m-R^{10}$ steht, worin

n für 1 oder 2 und

m für 0, 1 oder 2 stehen und

$R^{10}$ für gegebenenfalls durch Halogen substituiertes $C_1$-$C_4$-Alkyl, $C_3$-$C_5$-Alkenyl, $C_3$-$C_5$-Alkinyl oder $C_3$-$C_6$-Cycloalkyl, für gegebenenfalls durch Halogen, Cyano, Nitro, Trifluormethyl, Trifluormethoxy, Trifluormethylthio, $C_1$-$C_4$-Alkyl oder $C_1$-$C_4$-Alkoxy substituiertes Phenyl, Benzyl oder Phenylethyl oder für den Rest

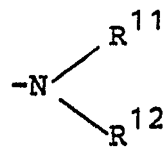

steht, worin

$R^{11}$ für $C_1$-$C_4$-Alkyl, $C_3$-$C_5$-Alkenyl, $C_3$-$C_5$-Alkinyl, $C_3$-$C_6$-Cycloalkyl oder Benzyl steht und

$R^{12}$ für $C_1$-$C_4$-Alkyl, $C_3$-$C_5$-Alkenyl, $C_3$-$C_5$-Alkinyl, $C_3$-$C_6$-Cycloalkyl, Benzyl, Phenylethyl, Halogencarbonyl, Formyl, $C_1$-$C_4$-Alkyl-carbonyl, $C_1$-$C_4$-Alkoxy-carbonyl, $C_1$-$C_4$-Alkoxyphenoxy-carbonyl, $C_3$-$C_5$-Alkinoxy-carbonyl, $C_3$-$C_5$-Alkenoxycarbonyl, $C_1$-$C_4$-Alkylthiocarbonyl, $C_1$-$C_4$-Alkyl-amino-carbonyl, $C_1$-$C_4$-Alkyl-hydroxylamino-carbonyl, $C_1$-$C_{10}$-Alkyl-phenoxycarbonyl, Di-$C_1$-$C_4$-alkyl-amino-carbonyl, Phenylthiocarbonyl, Phenoxycarbonyl, 2,3-Dihydro-2,2-dimethyl-7-benzofuranyloxycarbonyl, für gegebenenfalls durch Halogen, Cyano, Nitro, Trifluormethyl, $C_1$-$C_{10}$-Alkyl oder $C_1$-$C_4$-Alkoxy substituiertes Phenylsulfenyl, Phenylsulfinyl, Phenylsulfonyl oder Phenyl steht, oder für den Rest

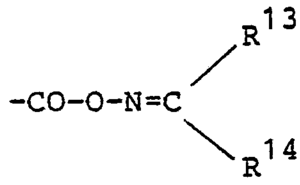

steht, worin

$R^{13}$ die oben für $R^8$ angegebene Bedeutung und

$R^{14}$ die oben für $R^9$ angegebene Bedeutung hat,

wobei ferner im Rest 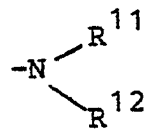 die Reste $R^{11}$ und $R^{12}$ zusammen für eine gegebenenfalls durch Sauerstoff oder Schwefel unterbrochene Kohlenwasserstoffkette mit 3 bis 8 Kohlenstoffatomen stehen, worin weiter $R^{10}$ auch für den gleichen Rest stehen kann, an den der Rest $-S_n(O)_m-R^{10}$ gebunden ist.

Als Wirkstoffkomponenten ganz besonders bevorzugt sind Carbamidsäureester der Formel (IV), in welcher

$R^4$ für gegebenenfalls durch $C_1$-$C_4$-Alkyl, $C_2$-$C_4$-Alkenyl, $C_1$-$C_4$-Alkoxy, $C_1$-$C_4$-Alkoxy-methyl, $C_1$-$C_4$-Alkylthio, $C_1$-$C_4$-Alkylthio-methyl, $C_1$-$C_4$-Alkylamino, Di-($C_1$-$C_4$-alkyl)-amino, Di-($C_3$-$C_4$-alkenyl)-amino, Halogen, Dioxolanyl, Methylendioxy und/oder durch den Rest $-N=CH-N(CH_3)_2$ substituierte Reste aus der Reihe Phenyl, Naphthyl, 2,3-Dihydro-7-benzofuranyl, Pyrazolyl oder Pyrimidinyl steht, oder in welcher

$R^4$ für einen Alkylidenaminorest der Formel (IVa)

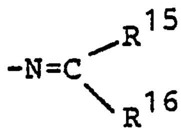

(IVa)

steht, in welcher

$R^{15}$ und $R^{16}$ die oben für $R^8$ bzw. $R^9$ angegebene Bedeutung haben, und

$R^5$ für $C_{1-4}$-Alkyl steht und

$R^6$ für Wasserstoff oder $C_1$-$C_4$-Alkyl (vorzugsweise für Wasserstoff) steht.

Als Beispiele für die Carbamidsäureester der Formel (IV) seien genannt: 2-Methyl-phenyl-, 2-Ethyl-phenyl-, 2-iso-Propyl-phenyl-, 2-sec-Butyl-phenyl-, 2-Methoxy-phenyl-, 2-Ethoxy-phenyl-, 2-iso-Propoxy-phenyl-, 4-Methyl-phenyl-, 4-Ethyl-phenyl-, 4-n-Propyl-phenyl-, 4-Methoxy-phenyl-, 4-Ethoxy-phenyl-, 4-n-Propoxy-phenyl-, 3,4,5-Trimethyl-phenyl-, 3,5-Dimethyl-4-methylthio-phenyl-, 3-Methyl-4-dimethylaminophenyl-, 2-Ethylthiomethyl-phenyl-, 1-Naphthyl-, 2,3-Dihydro-2,2-dimethyl-7-benzofuranyl, 2,3-(Dimethyl-methylendioxy)-phenyl-, 2-(4,5-Dimethyl-1,3-dioxolan-2-yl)-phenyl-, 1-Methylthio-ethyliden-amino-, 2-Methyl-thio-2-methylpropylidenamino-, 1-(2-Cyano-ethylthio)-ethylidenamino- und 1-Methylthiomethyl-2,2-dimethyl-propylidenamino-N-methyl-carbamidsäureester.

Die synergistische Wirkung der Verbindungen der allgemeinen Formel (I) zeigt sich weiter bevorzugt bei

2) Carbonsäureestern der Formel (V)

$$R^{17}\text{-CO-O-}\underset{}{\overset{\displaystyle R^{18}\atop |}{CH}}\text{-}R^{19} \qquad (V)$$

in welcher

$R^{17}$ für einen offenkettigen oder cyclischen Alkylrest steht, der gegebenenfalls substituiert ist durch Halogen, Alkyl, Cycloalkyl, durch gegebenenfalls durch Halogen, Alkyl und/oder Alkoxy substituiertes Alkenyl, durch Phenyl oder Styryl, welche gegebenenfalls durch Halogen, gegebenenfalls Halogen-substituierte Reste aus der Reihe Alkyl, Alkoxy, Alkylendioxy und/oder Alkylthio substituiert sind, durch spirocyclisch verknüpftes, gegebenenfalls Halogen-substituiertes Cycloalk(en)yl, welches gegebenenfalls benzannelliert ist, in welcher weiter

$R^{18}$ für Wasserstoff, Alkyl, Halogenalkyl, Alkenyl, Alkinyl oder Cyano steht, und

$R^{19}$ für einen gegebenenfalls substituierten Alkyl- oder Arylrest oder für einen Heterocyclus steht, oder zusammen mit $R^{18}$ und dem Kohlenstoffatom, an das beide Reste gebunden sind, einen Cyclopentenonring bildet.

Ganz besonders als Wirkstoffkomponenten bevorzugt sind Carbonsäureester der Formel (V), in welcher

$R^{17}$ für den Rest

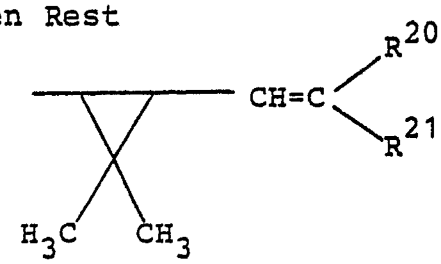

steht, worin

$R^{20}$ für Wasserstoff, Methyl, Fluor, Chlor oder Brom und

$R^{21}$ für Methyl, Fluor, Chlor, Brom, $C_1$-$C_2$-Fluoralkyl oder $C_1$-$C_2$-Chlorfluoralkyl oder für gegebenenfalls durch Halogen und/oder gegebenenfalls Halogen-substituierte Reste der Reihe $C_1$-$C_4$-Alkyl, $C_1$-$C_4$-Alkoxy, $C_1$-$C_4$-Alkylthio und/oder $C_1$-$C_2$-Alkylendioxy substituiertes Phenyl steht, oder worin beide Reste $R^{20}$ und $R^{21}$ für $C_2$-$C_5$-Alkandiyl (Alkylen) stehen; oder in welcher

$R^{17}$ für den Rest $-\underset{R^{23}}{\underset{|}{CH}}-R^{22}$ steht, worin

$R^{22}$ für gegebenenfalls durch Halogen und/oder durch gegebenenfalls Halogen-substituierte Reste der Reihe $C_1$-$C_4$-Alkyl, $C_1$-$C_4$-Alkoxy, $C_1$-$C_4$-Alkylthio oder $C_1$-$C_2$-Alkylendioxy substituiertes Phenyl steht und

$R^{23}$ für Isopropyl oder Cyclopropyl steht;

oder in welcher

$R^{17}$ für einen der Reste

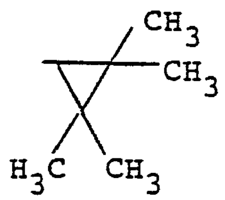

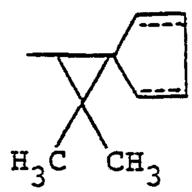

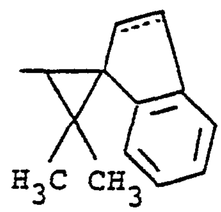

wobei die gepunkteten Linien mögliche Doppelbindungen andeuten sollen, oder für Methyl steht, und in welcher weiter

$R^{18}$ für Wasserstoff, $C_1$-$C_4$-Alkyl, $C_1$-$C_4$-Halogenalkyl, Cyano oder Ethinyl steht und

$R^{19}$ gegebenenfalls durch Halogen substituierte Reste der Reihe Phenyl, Furyl oder Tetrahydrophthalimido steht, wobei diese Reste ihrerseits substituiert sein können durch gegebenenfalls durch Halogen und/oder durch einen gegebenenfalls Halogen-substituierten Rest der Reihe $C_1$-$C_4$-Alkyl, $C_2$-$C_4$-Alkenyl, $C_1$-$C_4$-Alkoxy, $C_2$-$C_4$-Alkenoxy, $C_1$-$C_4$-Alkylthio, $C_1$-$C_2$-Alkylendioxy, Phenoxy und/oder Benzyl, wobei für $R^{19}$ vorzugsweise Pentafluorphenyl, 3,4-Dichlorphenyl, Phenoxyphenyl, welches in einem oder beiden Phenylringen durch Halogen substituiert sein kann oder für Tetrahydrophthalimido steht.

Weiter sind die natürlich vorkommenden Pyrethroide (wie Pyrethreum) als Carbonsäureester der Formel (V) besonders bevorzugt.

Als Beispiele für die Carbonsäureester der Formel (V) seien genannt:

Essigsäure-(2,2,2-trichlor-1-(3,4-dichlor-phenyl)-ethyl)-ester, 2,2-Dimethyl-3-(2-methyl-propen-1-yl)-cyclopropan-carbonsäure-(3,4,5,6-tetrahydro-phthalimido-methyl)-ester, 2,2-Dimethyl-3-(2,2-dichlor-vinyl)-cyclopropan-carbonsäure-(3-phenoxy-benzyl)-ester, 2,2-Dimethyl-3-(2,2-dichlorvinyl)-cyclopropan-carbonsäure-(α-cyano-3-phenoxy-benzyl)-ester, 2,2-Dimethyl-3-(2,2-dichlorvinyl)-cyclopropancarbonsäure-(α-cyano-4-fluor-3-phenoxy-benzyl)-ester, 2,2-Dimethyl-3-(2,2-dichlorvinyl)-cyclopropancarbonsäure-(pentafluor-benzyl)-ester, 2,2-Dimethyl-3-(2,2-dibromvinyl)-cyclopropancarbonsäure-(α-cyano-3-phenoxy-benzyl)-ester und 3-Methyl-2-(4-chlor-phenyl)-butansäure-(α-cyano-3-phenoxy-benzyl)-ester.

Weiter zeigt sich die synergistische Wirkung der Verbindungen der allgemeinen Formel (I) bevorzugt bei

3) Phosphorsäure- und Phosphonsäureestern der allgemeinen Formel (VI)

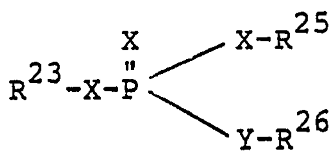

(VI)

in welcher

X jeweils für O oder S steht und

Y für O, S, -NH- oder für eine direkte Bindung zwischen dem zentralen P-Atom und $R^{26}$ steht und

$R^{24}$ und $R^{25}$ gleich oder verschieden sind und für gegebenenfalls substituiertes Alkyl oder Aryl stehen,

$R^{26}$ für Wasserstoff gegebenenfalls substituiertes Alkyl, Aryl, Heteroaryl, Aralkyl, Alkenyl, Dioxanyl oder einen Oximrest oder für den gleichen Rest steht, an den es gebunden ist.

Besonders bevorzugt sind Phosphorsäure- und Phosphonsäureester der Formel (VI), in welcher

$R^{24}$ und $R^{25}$ gleich oder verschieden sind und für $C_1$-$C_4$-Alkyl oder Phenyl stehen,

$R^{26}$ für Wasserstoff, Alkyl mit 1 bis 4 Kohlenstoffatomen steht, das gegebenenfalls durch Halogen, Hydroxyl, Cyano, gegebenenfalls Halogen-substituiertes Phenyl, Carbamoyl, Alkylsulfonyl, Alkylsulfinyl, Alkylcarbonyl, Alkoxy, Alkylmercapto,

Alkoxycarbonyl, Alkylaminocarbonyl, letztere mit jeweils bis zu 6 Kohlenstoffatomen, substituiert ist, für Alkenyl mit bis zu 4 Kohlenstoffatomen, das gegebenenfalls durch Halogen, gegebenenfalls Halogen-substituiertes Phenyl oder $C_1$-$C_4$-Alkoxycarbonyl substituiert ist, oder für den Rest der allgemeinen Formel (VIa)

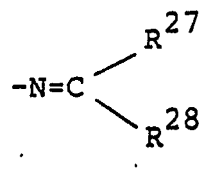

(VIa)

wobei $R^{27}$ und $R^{28}$ die oben für $R^8$ bzw. $R^9$ angegebene Bedeutung besitzen, oder für Cyano oder Phenyl stehen, und in welcher

$R^{26}$ ferner für Dioxanyl, das durch denselben Rest substituiert ist, an den $R^{25}$ gebunden ist, oder $R^{26}$ für den gleichen Rest, an den es gebunden ist, oder $R^{26}$ für Phenyl, das gegebenenfalls durch Methyl, Nitro, Cyano, Halogen und/oder Methylthio substituiert ist steht und $R^{26}$ außerdem besonders bevorzugt für gegebenenfalls durch $C_1$-$C_4$-Alkoxy, $C_1$-$C_4$-Alkylthiomethyl, $C_1$-$C_4$-Alkyl und/oder Halogen-substituierte heteroaromatische Reste, wie Pyridinyl, Chinolinyl, Chinoxalinyl, Pyrimidinyl oder Benzo-1,2,4-triazinyl steht.

Im einzelnen seien genannt:

O,O-Dimethyl- bzw. O,O-Diethyl-O-(2,2-dichlor- bzw. 2,2-dibromvinyl)-phosphorsäureester,
O,O-Diethyl-O-(4-nitro-phenyl)-thionophosphorsäureester
O,O-Dimethyl-O-(3-methyl-4-methylthio-phenyl)-thionophosphorsäureester
O,O-Dimethyl-O-(3-methyl-4-nitro-phenyl)-thionophosphorsäureester,
O-Ethyl-S-n-propyl-O-(2,4-dichlorphenyl)-thionophosphorsäureester,
O-Ethyl-S-n-propyl-O-(4-methylthio-phenyl)-thionophosphorsäureester,
O,O-Dimethyl-S-(4-oxo-1,2,3-benzothriazin(3)yl-methyl)-thionothiolphosphorsäureester,
O-Methyl-O-(2-iso-propyl-6-methoxy-pyrimidin(4)yl)-thionomethanphosphonsäureester,
O,O-Diethyl-O-(2-iso-propyl-6-methyl-pyrimidin(4)yl)-thionophosphorsäureester,
O,O-Diethyl-O-(3-chlor-4-methyl-cumarin(7)yl)-thionophosphorsäureester,
O,O-Dimethyl-2,2,2-trichlor-1-hydroxy-ethan-phosphonsäureester,
O,O-Dimethyl-S-(methylaminocarbonyl-methyl)-thionophosphorsäureester.

Weiterhin zeigt sich die synergistische Wirkung der Verbindungen der allgemeinen Formel (I) bevorzugt bei

4) Halogen(cyclo)-alkanen, wie z.B. Hexachlorcyclohexan, 1,1,1-Trichlor-2,2-bis-(4-chlorphenyl)-ethan, 1,1,1-Trichlor-2,2-bis-(4-methoxyphenyl)-ethan und 1,1-Dichlor-2,2-bis-(4-ethylphenyl)-ethan.

Die Gewichtsverhältnisse der Synergisten und Wirkstoffe können in einem relativ großen Bereich variiert werden. Im allgemeinen werden die als Synergisten verwendeten Verbindungen der Formel (I) mit den übrigen Wirkstoffen in Mischungsverhältnissen zwischen 1 : 100 und 100 : 1, vorzugsweise zwischen 1 : 5 und 5 : 1 (Gewichtsteile) eingesetzt.

Die erfindungsgemäßen Wirkstoffkombinationen besitzen nicht nur eine schnelle knock-down-Wirkung, sondern bewirken auch die nachhaltige Abtötung der tierischen Schädlinge, insbesondere von Insekten und Milben, die in der Landwirtschaft, in Forsten, im Vorrats- und Materialschutz sowie im Hygienebereich vorkommen. Sie sind gegen normal sensible und resistente Arten sowie gegen alle oder einzelne Entwicklungsstadien wirksam.

Zu den tierischen Schädlingen, welche unter Verwendung der Verbindungen der Formel (I) bekämpft werden können, gehören beispielsweise:

Aus der Ordnung der Isopoda z.B. Oniscus asellus, Porcellio scaber.
Aus der Ordnung der Thysanura z.B. Lepisma saccharina.

Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Schistocerca gregaria.

Aus der Ordnung der Dermaptera z.B. Forficula auricularia.

Aus der Ordnung der Isoptera z.B. Reticulitermes spp.

Aus der Ordnung der Anoplura z.B. Pediculus humanus corporis, Haematopinus spp., Linograthus spp.

Aus der Ordnung der Mallophaga z.B. Trichodectes spp., Damalinea spp.

Aus der Ordnung der Heteroptera z.B. Cimex lectularius, Rhodnius prolixus, Triatoma spp.

Aus der Ordnung der Homoptera z.B. Myzus spp., und Psylla spp.

Aus der Ordnung der Lepidoptera z.B. Ephestia kuehniella und Galleria mellonella.

Aus der Ordnung der Coleoptera z.B. Anobium punctatum, Rhizopertha dominica, Bruchidius obtectus, Acanthoscelides obtectus, Hylotrupes bajulus, Oryzaephilus surinamensis, Sitophilus spp., Dermestes spp., Trogoderma spp., Anthrenus spp., Attagenus spp., Lyctus spp., Ptinus spp., Niptus hololeucus, Gibbium psylloides, Tribolium spp. und Tenebrio molitor.

Aus der Ordnung der Hymenoptera z.B. Lasius spp., Monomorium pharaonis, Vespa spp.

Aus der Ordnung der Diptera z.B. Aëdes spp., Anopheles spp., Culex spp., Drosophila melanogaster, Musca spp., Fannia spp., Calliphora erythrocephala, Lucilia spp.,

Chrysomyia spp., Gastrophilus spp., Hyppobosca spp., Stomoxys spp., Oestrus spp., Hypderma spp. und Tabanus spp.
Aus der Ordnung der Siphonaptera z.B. Xenopsylla cheopis, Ceratophyllus spp.
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Boophilus spp., Rhipicephalus spp., Amblyomma spp., Hyalomma spp., Ixodes spp., Psoroptes spp., Chorioptes spp., Sarcoptes spp.

Die Wirkstoffkombinationen aus den Verbindungen der Formel (I) und den übrigen Wirkstoffen können in die üblichen Formulierungen übergeführt werden, wie Lösungen, Emulsionen, Spritzpulver, Suspensionen, Pulver, Stäubemittel, Schäume, Pasten, lösliche Pulver, Aerosole, Suspensions-Emulsionskonzentrate, Wirkstoffimprägnierte Natur- und synthetische Stoffe, Feinstverkapselungen in polymeren Stoffen, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffgemische mit Streckmitteln, also flüssigen Lösungsmitteln, unter Druck stehenden verflüssigten Gasen und/oder festen

Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glykol, sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe: natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate: gebrochene und fraktionierte

natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel: nichtionogene und anionische Emulgatoren, wie Polyoxyethylen-Fettsäure-Ester, Polyoxyethylen-Fettalkohol-Ether, z.B. Alkylaryl-polyglykol-ether, Alkylsulfonate; als Dispergiermittel: z.B. Lignin, Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe, und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gew.-% Wirkstoffkombination, vorzugsweise zwischen 0,5 und 90 %.

Die Anwendung der erfindungsgemäßen Wirkstoffkombinationen erfolgt in Form ihrer handelsüblichen Formulierungen und/oder den aus diesen Formulierungen bereiteten Anwendungsformen.

Der gesamte Wirkstoffgehalt (einschließlich Synergist) der aus den handelsüblichen Formulierungen bereiteten Anwendungsformen kann in weiten Bereichen variieren. Die Wirkstoffkonzentration der Anwendungsformen kann von 0,001 bis zu 100 Gew.-% Wirkstoffkombination, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffkombinationen durch eine hervorragende Residualwirkung auf Holz und Ton sowie durch eine gute Alkalistabilität auf gekälkten Unterlagen aus.

Anhand der folgenden biologischen Beispiele soll die Wirksamkeit der erfindungsgemäß verwendbaren Verbindungen der Formel (I) erläutert werden:

Angewendete Testmethode:

$LT_{100}$-Test

Testtiere: Musca domestica, Stamm Weymanns (gegen Carbamidsäureester und gegen P-Ester resistent)

Lösungsmittel: Aceton

Von den Wirkstoffen, Synergisten und Gemischen aus Wirkstoffen und Synergisten werden Lösungen hergestellt und 2,5 ml davon in Petrischalen auf Filterpapierscheiben von 9,5 cm Durchmesser pipetiert. Das Filterpapier saugt die Lösungen auf. Die Petrischalen bleiben so lange offen stehen, bis das Lösungsmittel vollständig verdunstet ist. Anschließend gibt man 25 Testtiere in die Petrischalen und bedeckt sie mit einem Glasdeckel.

Der Zustand der Tiere wird bis zu 6 Stunden fortlaufend kontrolliert.
Es wird diejenige Zeit ermittelt, die für eine 100 %ige knock down-Wirkung erforderlich ist. Wird die $LT_{100}$ nach 6 Stunden nicht erreicht, wird der % Satz der knock down gegangenen Testtiere festgestellt.

Wirkstoffe, Synergisten, die Konzentrationen der Wirkstoffe, Synergisten und Gemische sowie ihre Wirkungen gehen aus den nachfolgenden Tabellen hervor.

Tabelle I

Beispiele für erfindungsgemäß verwendbare Arthropodizide

A.) 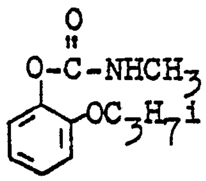 (Propoxur)

B.) 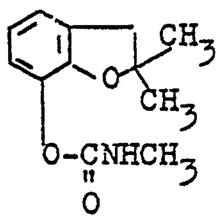 (Carbofuran)

C.) 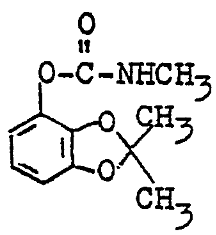 (Bendiocarb)

D.) 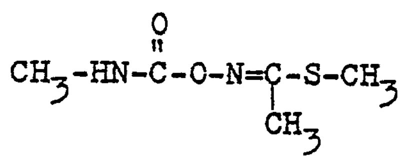 (Methomyl)

E.) Pyrethrine natürlicher Herkunft als 25 %iger Extrakt

F.) 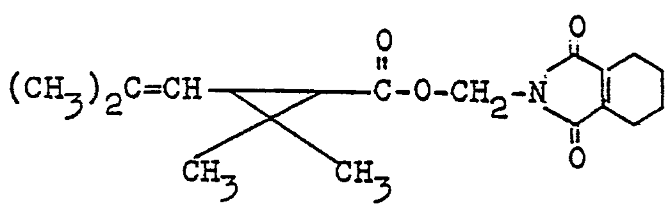 (Tetramethrin)

G.) 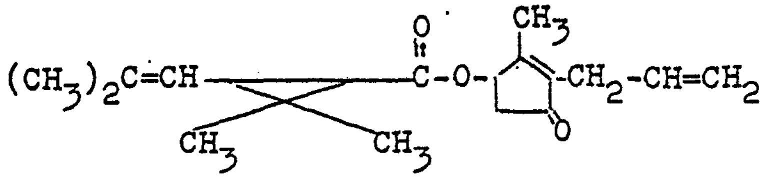 (Bioallethrin)

L.) 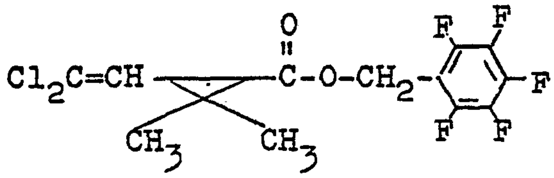 (Fenfluthrin)

M.) 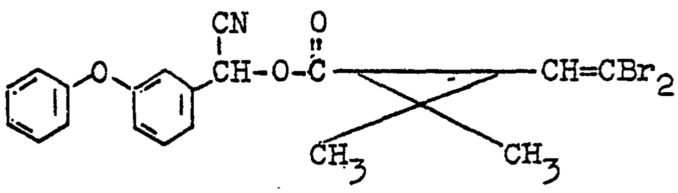 (Decamethrin)

N.) 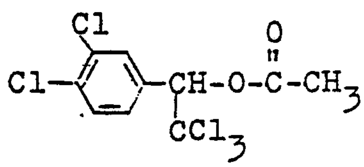 (Penfenate)

O.) 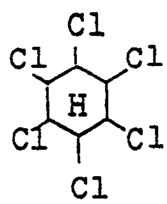 (γ-Hexachlorcyclohexan)

P.) 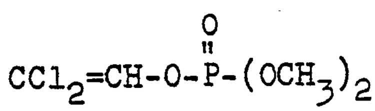 (DDVP)

Q.) 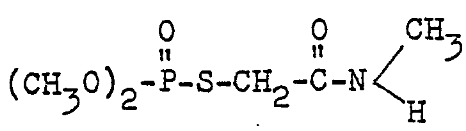 (Omethoat)

R.) 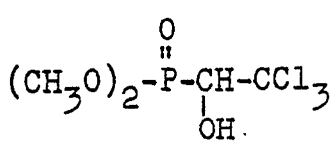 (Trichlorfon)

S.) 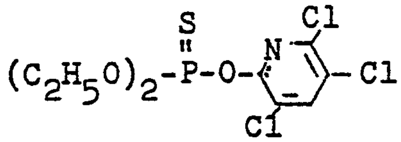 (Chlorpyrifos)

T.) 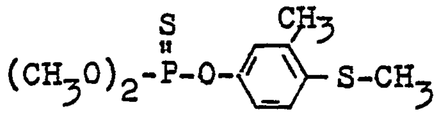 (Fenthion)

U.) 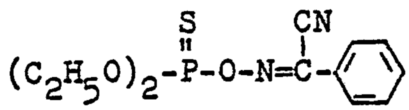 (Phoxim)

V.) 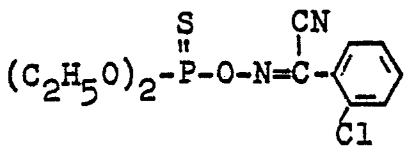 (Chlorphoxim)

W.) 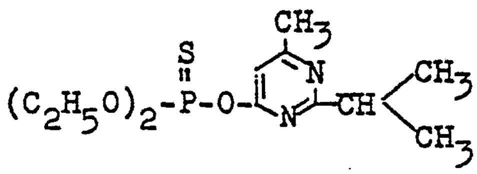 (Diazinon)

X.) 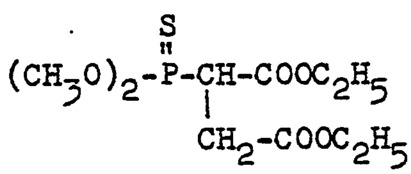 (Malathion)

Tabelle II

Beispiele von erfindungsgemäß verwendbaren Synergisten

1.) 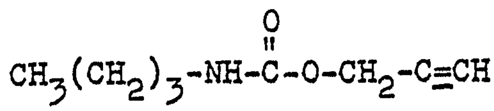

2.) 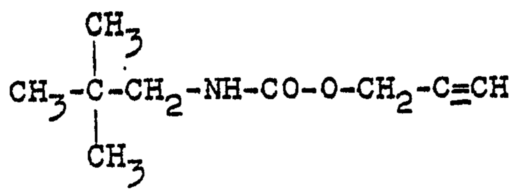

3.) 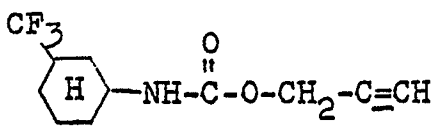

4.) Standard-Synergist:
Piperonylbutoxid
(Handelsprodukt)

| Wirkstoff Kennbuchstabe (vgl. Tabelle I) | + Synergist Nr. (vgl. Tabelle II) | Konzentrationen in % Wirkstoff | + Synergist | LT 100 nach Minuten bzw. % nach 360' |
|---|---|---|---|---|
| A) | - | 1,0 | - | 360'=10 % |
| B) | - | 1,0 | - | 360'=25 % |
| C) | - | 1,0 | - | 360'= 0 % |
| D) | - | 0,2 | - | 360'=95 % |
| E) | - | 0,04 | - | 360'=65 % |
| F) | - | 0,04 | - | 360'= 0 % |
| G) | - | 0,04 | - | 90' |
| L) | - | 0,00032 | - | 360'=50 % |
| M) | - | 0,008 | - | 75' |
| N) | - | 1,0 | - | 360'=50 % |
| O) | - | 0,04 | - | 105' |
| P) | - | 0,008 | - | 210' |
| Q) | - | 0,04 | - | 360'=45 % |
| R) | - | 1,0 | - | 360' |
| S) | - | 1,0 | - | 360'=45 % |
| T) | - | 0,2 | - | 360'=90 % |
| U) | - | 1,0 | - | 360'=90 % |
| V) | - | 1,0 | - | 360'=20 % |
| W) | - | 0,04 | - | 360'=65 % |
| X) | - | 1,0 | - | 360'=45 % |
| - | 1. | - | 0,04 | 180' |
| - | 2. | - | 0,2 | 240' |
| - | 3. | - | 0,2 | 360'=20 % |
| - | 4. | - | 1,0 | 360'= 0 % |
| A | +1 | 0,04 | 0,04 | 120' |
| A | +2 | 0,04 | 0,04 | 90' |
| A | +3 | 0,04 | 0,04 | 90' |
| A | +4 | 0,04 | 0,04 | 360'=80 % |

| Wirkstoff Kennbuchstabe (vgl. Tabelle I) | + Synergist Nr. (vgl. Tabelle II) | Konzentrationen in % Wirkstoff | + Synergist | LT 100 nach Minuten bzw. % nach 360' |
|---|---|---|---|---|
| B | + 2 | 0,008 | + 0,008 | 105' |
| B | + 4 | 0,2 | + 0,2 | 360'=90 % |
| C | + 2 | 0,008 | + 0,008 | 150' |
| C | + 4 | 0,2 | + 0,2 | 360'=35 % |
| D | + 2 | 0,04 | + 0,04 | 180' |
| D | + 4 | 0,04 | + 0,04 | 360'=20 % |
| E | + 2 | 0,04 | + 0,04 | 75' |
| F | + 2 | 0,04 | + 0,04 | 150' |
| F | + 4 | 0,04 | + 0,04 | 360'=95 % |
| G | + 2 | 0,04 | + 0,04 | 60' |
| G | + 4 | 0,04 | + 0,04 | 60' |
| L | + 2 | 0,00032 | + 0,00032 | 360' |
| L | + 4 | 0,00032 | + 0,00032 | 360'=80 % |
| M | + 2 | 0,008 | + 0,008 | 60' |
| M | + 4 | 0,008 | + 0,008 | 75' |
| N | + 1 | 0,04 | + 0,04 | 120' |
| N | + 2 | 0,2 | + 0,2 | 120' |
| N | + 3 | 0,04 | + 0,04 | 90' |
| N | + 4 | 0,2 | + 0,2 | 360'=80 % |
| O | + 2 | 0,04 | + 0,04 | 60' |
| O | + 4 | 0,04 | + 0,04 | 105' |
| P | + 2 | 0,008 | + 0,008 | 90' |
| P | + 4 | 0,008 | + 0,008 | 210' |
| Q | + 2 | 0,04 | + 0,04 | 150' |
| Q | + 4 | 0,04 | + 0,04 | 360' |

| Wirkstoff Kennbuchstabe (vgl. Tabelle I) | + Synergist Nr. (vgl. Tabelle II) | Konzentrationen in % Wirkstoff | + Synergist | LT 100 nach Minuten bzw. % nach 360' |
|---|---|---|---|---|
| R | + 2 | 1,0 | + 1,0 | 60' |
| R | + 4 | 1,0 | + 1,0 | 360' |
| S | + 2 | 0,2 | + 0,2 | 360' |
| S | + 4 | 1,0 | + 1,0 | 360'=85 % |
| T | + 2 | 0,04 | + 0,04 | 180' |
| T | + 4 | 0,2 | + 0,2 | 360'=80 % |
| U | + 2 | 0,04 | + 0,04 | 360' |
| U | + 4 | 1,0 | + 1,0 | 360'=60 % |
| V | + 2 | 0,04 | + 0,04 | 180' |
| V | + 4 | 1,0 | + 1,0 | 360'=95 % |
| W | + 2 | 0,04 | + 0,04 | 180' |
| W | + 4 | 0,04 | + 0,04 | 360'=70 % |
| X | + 2 | 0,2 | + 0,2 | 180' |
| X | + 4 | 1,0 | + 1,0 | 360'=30 % |

Die Herstellung der erfindungsgemäßen Verbindungen der allgemeinen Formel (I) (bzw. (Ia)) soll durch die folgenden Herstellungsbeispiele erläutert werden:

## Herstellungsbeispiele

$(CH_3)_3C-CH_2-NH-CO-O-CH_2-C{\equiv}CH$ (Beispiel 2)

N-(2,2-Dimethyl-propyl)-carbamidsäure-O-2-propinylester

Variante mit Verdünnungsmittel:

11,3 g N-(2,2-Dimethyl-propyl)-isocyanat werden in 60 ml Aceton gelöst und 10 mg Diazabicyclooctan (DABCO) (= Triethylendiamin) zugesetzt. Zu diesem Reaktionsgemisch tropft man bei Raumtemperatur 5,6 g 2-Propinyl-alkohol zu. Nach einstündigem Erhitzen des Gemisches zum Sieden läßt man erkalten. Nach dem Einengen wird der gebildete Feststoff (N,N'-Dineopentylharnstoff, Fp. 100°C) abfiltriert und verworfen. Das zurückbleibende Öl im Hochvakuum vom restlichen Lösungsmittel befreit. Man erhält 11 g N-Neopentyl-carbamidsäure-propargyl-ester in Form eines farblosen Öls (65 % der Theorie Ausbeute) $n_D^{20}$:1,4561.

Variante ohne Verdünnungsmittel:

Zu 22,6 g (N-(2,2-Dimethyl-propyl)-isocyanat, die 10 mg Diazabicyclooctan (DABCO) (= Triethylendiamin) enthalten, werden bei 60°C 11,2 g Propinyl-alkohol zugetropft. Nach 1 bis 2 Stunden zeigt das IR-Spektrum einer Probe keine NCO-Bande mehr. Nach Destillation bei 85°C / 0,03 mbar erhält man 32.0 g (95,4% der Theorie) der obigen Verbindung mit einer Reinheit von 99,7% (Gaschromatogramme).

Die übrigen erfindungsgemäß verwendbaren Verbindungen werden in analoger Weise erhalten:

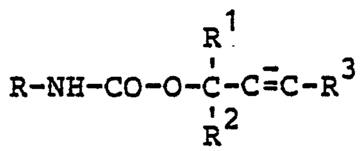

| Beispiel Nr. | R | $R^1$ | $R^2$ | $R^3$ | Brechungsindex $n_D^{20}$ (bzw. Fp (°C)) |
|---|---|---|---|---|---|
| 1 | $CH_3(CH_2)_3-$ | H | H | H | 1,4560 |
| 3 | 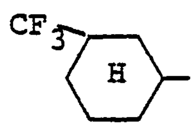 | H | H | H | 1,4450 |
| 4 | $(CH_3)_3C-CH(CH_3)-$ | H | H | H | 1,4642 |
| 5 | $(CH_3)_2CH-CH_2-$ | H | H | H | 1,4576 |
| 6 | $(C_2H_5)_2CH-$ | H | H | H | 1,4572 |
| 7 | $NC-(CH_2)_5-$ | H | H | H | (Fp) 54-56 |
| 8 | $Cl-(CH_2)_6-$ | H | H | H | 1,4859 |

Patentansprüche

1. Schädlingsbekämpfungsmittel, welche eine Wirkstoffkombination aus wenigstens einem gegen Arthropoden wirksamen Wirkstoff und einem Synergisten enthalten, dadurch gekennzeichnet, daß der Synergist ein Carbamidsäureester mit der allgemeinen Formel (I)

$$R-NH-CO-O-\underset{R^2}{\overset{R^1}{C}}-C\equiv C-R^3 \qquad (I)$$

in welcher

R für gegebenenfalls substituiertes Alkyl mit 4 oder mehr Kohlenstoffatomen oder gegebenenfalls substituiertes Cycloalkyl steht und

$R^1$, $R^2$ und $R^3$ gleich oder verschieden sind und einzeln für Wasserstoff oder für gegebenenfalls substituiertes Alkyl stehen,

darstellt.

2. Schädlingsbekämpfungsmittel gemäß Anspruch 1, wobei in Formel (I)

R für einen gegebenenfalls durch Alkoxy ($C_1$-$C_4$), Halogen, Cyano, Nitro und/oder Trifluormethyl substituierten Alkylrest mit 4-12 Kohlenstoffatomen oder für einen gegebenenfalls durch Alkoxy ($C_1$-$C_4$), Halogen, Cyano, Nitro und/oder Trifluormethyl substituierten Cycloalkylrest mit 4 bis 10 Kohlenstoffatomen steht und

$R^1$,$R^2$,$R^3$ gleich oder verschieden sind und einzeln für Wasserstoff oder für einen gegebenenfalls durch Halogen, Cyano, $C_1$-$C_4$-Alkoxy, $C_1$-$C_4$-Alkylthio oder Trifluormethyl substituierten Alkylrest mit 1-2 Kohlenstoffatomen stehen.

3. Schädlingsbekämpfungsmittel gemäß Anspruch 1, wobei in Formel (I)

R für gegebenenfalls durch Chlor oder Cyano substituiertes Alkyl mit 4 bis 6 Kohlenstoffatomen oder für gegebenenfalls durch Trifluormethyl substituiertes Cyclohexyl steht und

$R^1$,$R^2$ und $R^3$ für Wasserstoff stehen.

4. Verwendung der Schädlingsbekämpfungsmittel gemäß den Ansprüchen 1 bis 3 zur Bekämpfung von Schädlingen, insbesondere Arthropoden.

5. Verfahren zur Herstellung von Schädlingsbekämpfungsmitteln, dadurch gekennzeichnet, daß man eine Wirkstoffkombination aus wenigstens einem gegen Arthropoden wirksamen Wirkstoff und einem Synergisten der Formel (I) (gemäß Anspruch 1) mit den üblichen Streck- und Verdünnungsmitteln sowie gegebenenfalls Formulierhilfsstoffen, wie Emulgatoren, vermischt.

6. Verfahren zur Bekämpfung von Schädlingen, dadurch gekennzeichnet, daß man Schädlingsbekämpfungsmittel gemäß den Ansprüchen 1 bis 4 auf die Schädlinge und/oder ihren Lebensraum einwirken läßt.

7. Carbamidsäureester der allgemeinen Formel (Ia)

$$R-NH-CO-O-\underset{R^2}{\overset{R^1}{C}}-C\equiv C-R^3 \qquad (Ia)$$

in welcher

R für gegebenenfalls substituiertes Alkyl mit 4 oder mehr Kohlenstoffatomen oder gegebenenfalls substituiertes Cycloalkyl steht und

$R^1$, $R^2$ und $R^3$ gleich oder verschieden sind und einzeln für Wasserstoff oder für gegebenenfalls substituiertes Alkyl stehen,

ausgenommen die Verbindung in welcher R für n-Butyl und $R^1$, $R^2$ und $R^3$ für Wasserstoff stehen.

8. Carbamidsäureester gemäß Anspruch 7, wobei

R für Neopentyl oder für gegebenenfalls durch Trifluormethyl substituiertes Cyclohexyl steht und

$R^1$, $R^2$ und $R^3$ für Wasserstoff stehen.

9. Carbamidsäureester der Formel

$$(CH_3)_3C-CH_2-NH-CO-O-CH_2-C\equiv CH$$

10. Verfahren zur Herstellung von Carbamidsäureestern der allgemeinen Formel (Ia)

$$R-NH-CO-O-\underset{R^2}{\overset{R^1}{\underset{|}{\overset{|}{C}}}}-C\equiv C-R^3 \qquad \text{(Ia)}$$

in welcher

R für gegebenenfalls substituiertes Alkyl mit 4 oder mehr Kohlenstoffatomen oder gegebenenfalls substituiertes Cycloalkyl steht und

$R^1$, $R^2$ und $R^3$ gleich oder verschieden sind und einzeln für Wasserstoff oder für gegebenenfalls substituiertes Alkyl stehen,

ausgenommen die Verbindung in welcher R für n-Butyl und $R^1$,$R^2$ und $R^3$ für Wasserstoff stehen,

dadurch gekennzeichnet, daß man Alkylisocyanate der allgemeinen Formel (II)

$$R - NCO \qquad (II)$$

in welcher

R die eingangs erwähnte Bedeutung hat,

mit Alkinylalkoholen der allgemeinen Formel (III)

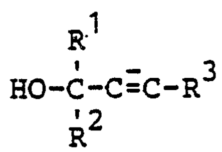

(III)

in welcher

$R^1$, $R^2$ und $R^3$ die eingangs erwähnte Bedeutung haben,

gegebenenfalls in Gegenwart eines aprotischen Verdünnungsmittels und gegebenenfalls in Gegenwart eines Katalysators bei Temperaturen zwischen 0 und 120°C umsetzt.

0101006

Europäisches Patentamt

# EUROPÄISCHER RECHERCHENBERICHT

Nummer der Anmeldung

EP 83 10 7633

| EINSCHLÄGIGE DOKUMENTE | | | KLASSIFIKATION DER ANMELDUNG (Int. Cl. 3) |
|---|---|---|---|
| Kategorie | Kennzeichnung des Dokuments mit Angabe, soweit erforderlich, der maßgeblichen Teile | Betrifft Anspruch | |
| D,X | BE-A- 633 594 (BASF) * Ansprüche; Seite 5, Beispiele * | 1-10 | C 07 C 125/065<br>A 01 N 47/12<br>A 01 N 47/18 |
| X | DE-C- 953 875 (CIBA) * Beispiele * | 7-10 | |
| X | JOURNAL OF MEDICINAL CHEMISTRY, Band 22, Nr. 6, Juni 1979, Seiten 746-748, American Chemical Society, Washington, D.C., US SAI-KEUNG CHIU u.a.: "Synthesis of imidazolidinediones and oxazolidinediones from cyclization of propargylureas and propargyl carbamates" * Seite 747 * | 7-10 | |
| | | | RECHERCHIERTE SACHGEBIETE (Int. Cl. 3) |
| X | CHEMICAL ABSTRACTS, Band 84, Nr. 25, Juni 1976, Seite 566, Nr. 180113e, Columbus, Ohio, US T. FRANCIS u.a.: "Carbamates and 2-oxazolidinones from tertiary alcohols and isocyanates" & CAN. J. CHEM. 1976, 54(1), 24-30 | 7-10 | C 07 C 125/00<br>A 01 N 47/00 |

Der vorliegende Recherchenbericht wurde für alle Patentansprüche erstellt.

| Recherchenort | Abschlußdatum der Recherche | Prüfer |
|---|---|---|
| DEN HAAG | 18-11-1983 | GAUTIER R.H.A. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82